Europäisches Patentamt

European Patent Office

Office européen des brevets

(11) Publication number: **0 128 765**
A2

# EUROPEAN PATENT APPLICATION

(21) Application number: 84303916.5

(22) Date of filing: 08.06.84

(51) Int. Cl.³: **A 61 B 1/00**

(30) Priority: 09.06.83 JP 103940/83

(43) Date of publication of application: 19.12.84
Bulletin 84/51

(84) Designated Contracting States: **DE FR GB IT NL SE**

(71) Applicant: **SUMITOMO ELECTRIC INDUSTRIES
LIMITED, No. 15, Kitahama 5-chome Higashi-ku,
Osaka-shi Osaka-fu (JP)**

(72) Inventor: **Hiramoto, Junichi c/o Osaka Works of
Sumitomo, Electric Industries, Ltd. 1-3,
Shimaya 1-chome, Konohana-ku Osaka (JP)**
Inventor: **Tsuno, Koichi c/o Osaka Works of Sumitomo,
Electric Industries, Ltd. 1-3, Shimaya 1-chome,
Konohana-ku Osaka (JP)**

(74) Representative: **Warren, Keith Stanley et al, BARON &
WARREN 18 South End Kensington, London W8 5BU
(GB)**

(54) Optical transmission channel.

(57) The present invention relates to the structure of an optical
transmission channel (13) for use in a fiberscope, an optical
fiber sensor or the like having an illuminating light transmission
channel, an information light transmission channel and various
kinds of auxiliary transmission channels. The optical transmis-
sion channel (13) is provided in its periphery with axially and
extending grooves for receiving an image fiber (10) and a tube
(12) for conducting fluid, the arrangement being such that the
transmission channel has an almost circular section when the
grooves are fitted with the image fiber and tube.

## OPTICAL TRANSMISSION CHANNEL

The present invention relates to an optical transmission channel for use in a fiberscope, an optical fiber sensor or the like having an illuminating light transmission channel, an information light transmission channel and various kinds of auxiliary transmission channels.

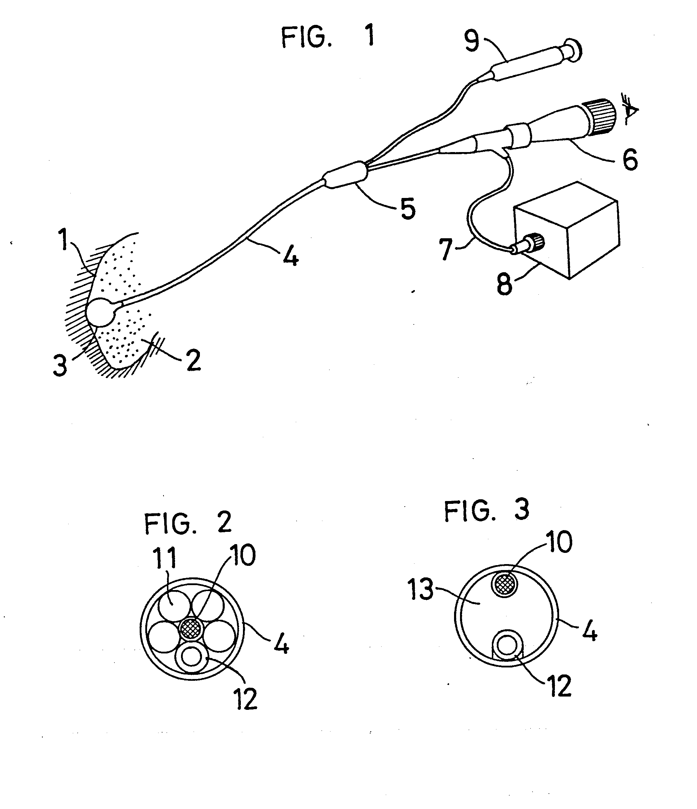

A conventional optical transmission channel is illustrated in Fig. 1 as incorporated in a fiberscope for heart and blood vessels and as disclosed in Japanese Laid-Open Patent No. 55-151936 (1980) entitled "Internal Observation Apparatus". A special mechanism is required for such observation since the heart and blood vessels are filled with opaque liquids - blood.

Referring to Fig. 1, a flexible cable portion 4 of a fiberscope comprising illuminating light transmission fiber optics and an information light transmission image fiber (image direct-transmission fiber optics) is provided with a transparent balloon 3 at the head thereof to enable observation of the internal wall 1 of a heart. The balloon 3 is flexible and when inflated, removes blood 2, which obstructs the field of view in front of the fiberscope, thereby enabling observation of the internal wall 1 of the heart.

6 is an image-receiving adaptor for observing transmitted images, 8 is a source of illuminating light, and 9 is a syringe for expanding and contracting the balloon 3.

The flexible cable portion 4 has a section as shown in Fig. 2. In the section, four pieces of illuminating light transmission plastic fiber 11, arranged around an image fiber 10, and a tube 12 for expanding and contracting the balloon 3 are housed within the flexible cable portion 4.

An optical transmission channel of the above described conventional construction has the following disadvantages:

(1) Large gaps are formed among the image fiber 10, to plastic fibers 11, the tube 12 and the like, so that the efficient utilisation of the sectional area of the flexible cable portion 4 is small because the image fiber 10, the plastic fibers 11, the tube 12 and the like have circular cross-sections and they are housed in a flexible cable portion 4 which is also of circular cross-section.

(2) The collecting and branching arrangements of the fibers and tube are expensive.

It is an object of the present invention to provide an optical transmission channel which alleviates the disadvantages incidental to conventional optical transmission channels and is capable of improving the above described utilisation of the cross-sectional area and facilitating the making of the collecting and branching arrangements of the fibers and tube.

In order that the invention may be more readily understood, reference will now be made to the accompanying drawings, in which:-

Fig. 1 is a diagram showing an example of the structure of a fiberscope for a heart and blood vessels,

Fig. 2 is a sectional view of a conventional fiberscope,

Fig. 3 is a sectional view of a fiberscope embodying an optical transmission channel according to the present invention,

Fig. 4 is a diagram showing the structure of an optical transmission channel according to the invention,

Fig. 5 is a diagram showing the assembly procedure for an optical transmission channel according to the invention,

Fig. 6 is a diagram showing a metallic mold for forming an optical transmission channel according to the invention, and

Fig. 7 is a sectional view through the mold shown in Fig. 6.

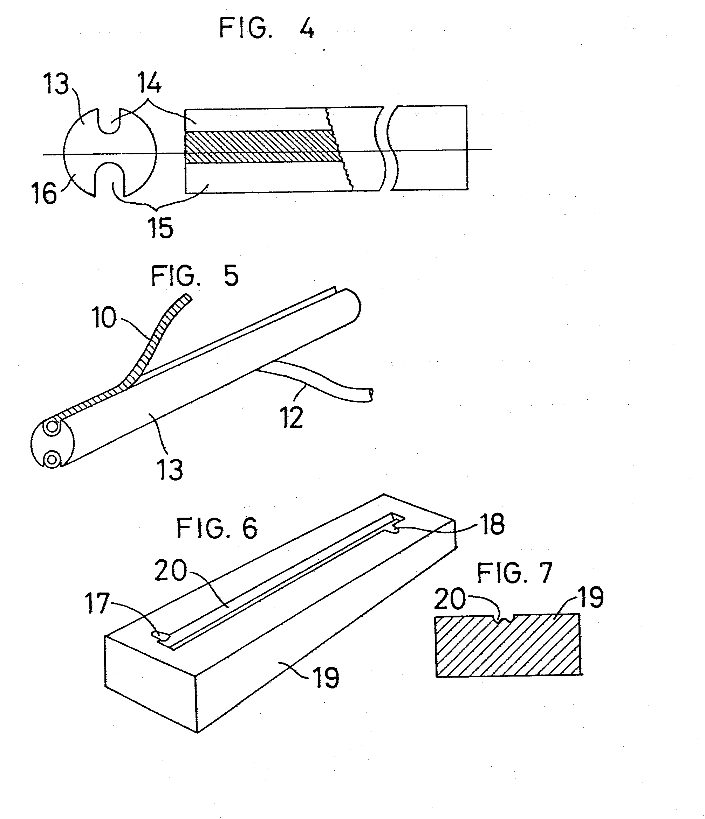

Referring nwo to Fig. 3, the section of a grooved optical transmission channel 13 according to the present invention accounts for most of the cross-sectional area of a flexible cable portion 4 in which it is housed, same for sections of an image fiber 10 and a tube 12. As shown in Fig. 4, this grooved optical transmission channel 13 is provided with a groove 14 for an image fiber and a groove 15 for a balloon inflation tube. These grooves are linear and are disposed in the circumference or periphery of the channel 13,parallel to the longitudinal axis thereof. As shown in Fig. 5, the grooves 14, 15 facilitate the collecting operation and branching operation of the image fiber and the tube 12.

The optical transmission channel can be produced in a two-segment type metallic mold 19 (one segment being omitted), as shown in Fig. 6. A groove 20 in the metallic mold segment has a section as shown in Fig. 7 and is filled with transparent material, such as PMMA (polymethylmethacrylate), PiBMA (polyisobutylmethacrylate), PnBMA (polynormalbutylmethacrylate), polystyrene, polycarbonate or silicic rubber, via a filling port 17 and an overflow port 18. In addition, it is necessary that the external surface of the resulting grooved optical transmission channel 13 be covered with a cad 16 (Fig.4) - a thin film of the materials having a refractive index smaller than that of the transparent materials by an immersion or dipping method or the like in order to control the optical loss resulting from damage produced in the assemblying operation.

With the present invention,

(1) the efficient utilisation of the cross-sectional area can be improved, thereby enabling the reduction in diameter of an optical transmission channel as a whole (being easily inserted into blood vessels and the like) provided that the sectional area of an illuminating light transmission channel is constant, and (2) the collecting and branching operations of the fibers and tube can be more easily carried out.

The grooved optical transmission channel according to the present invention can be used in such fields as (1) an illuminating light transmission channel of a fiberscope and (2) an optical transmission channel of various kinds of fiber optics sensors.

CLAIMS

1.      An optical transmission channel (13)
characterized by at least one axially extending
groove (14, 15) in the periphery thereof, the
section of the optical transmission channel being
substantially circular when the or each groove
is filled.

2.      An optical transmission channel according
to claim 1, characterized in that it is made from a
transparent material, such as, PMMA (polymethylmethacrylate), PiBMA (polyisobutylmethacrylate), PnBMA
(polynormalbutylmethacrylate) polystyrene, polycarbonate
and/or silicic rubber.

0128765

1/2

FIG. 1

FIG. 2

FIG. 3

FIG. 4

FIG. 5

FIG. 6

FIG. 7